# EUROPÄISCHE PATENTSCHRIFT

(11) **EP 0 518 883 B1**
(45) Veröffentlichungstag und Bekanntmachung des Hinweises auf die Patenterteilung: **23.11.1994**
(21) Anmeldenummer: 91904224.2
(22) Anmeldetag: 26.02.1991
(51) Int. Cl.: C07C 59/10, C08K 5/09, C08L 27/06

(54) **2,2-DIMETHYLOLPROPIONSÄUREMETALLSALZE UND IHRE VERWENDUNG ALS KUNSTSTOFFSTABILISATOREN**
2,2-DIMETHYLOLPROPRIONIC ACID AND METAL SALTS AND THEIR USE AS STABILIZERS IN PLASTICS
SELS METALLIQUES D'ACIDE 2,2-DIMETHYLOLPROPRIONIQUE ET LEUR UTILISATION COMME STABILISANTS DE MATIERES PLASTIQUES

(30) Priorität: 05.03.1990 DE 4006883
(43) Veröffentlichungstag der Anmeldung: 23.12.1992
(73) Patentinhaber: Henkel Kommanditgesellschaft auf Aktien, 40191 Düsseldorf (DE)
(72) Erfinder: ERWIED, Werner, D-4010 Hilden (DE); LANGE, Fritz, D-4300 Essen (DE); KRAMPITZ, Dieter, D-4050 Mönchengladbach 1 (DE); WORSCHECH, Kurt, D-2854 Loxstedt-Bexhövede (DE)
(86) Internationale Anmeldenummer: EP9100352
(87) Internationale Veröffentlichungsnummer: WO9113857

(56) Entgegenhaltungen:
- EP-A- 0 313 113
- DE-A- 3 534 947

## Beschreibung

Die Erfindung betrifft Metallsalze der 2,2-Dimethylolpropionsäure der allgemeinen Formel I

[CH₃ - C(CH₂OH)₂ - COO⁻]₂ M²⁺ (I)

in der M ein Metall aus der von Magnesium, Calcium und Zink gebildeten Gruppe bedeutet.

Bei Herstellung von Halbzeug und Fertigwaren aus Hart-PVC besteht neuerdings die Tendenz, die bisher üblichen Schwermetallstabilisatoren wie Blei- und Cadmiumverbindungen durch solche auf Calcium- und Zinkbasis zu ersetzen, vgl. Gächter/Müller, Kunststoff-Additive, 2. Ausgabe, 1983, Carl Hanser Verlag, München, Wien, Seite 222-224. Zink-stabilisierte PVC-Massen weisen jedoch den Nachteil des sogenannten "Zink-burning" auf, d.h. nach einer gewissen thermischen Belastung im Verlauf der Verarbeitung der Massen durch Kalandrieren oder dergleichen kann es zu einem plötzlichen Auftreten einer Schwarzverfärbung kommen. Zwar läßt sich dieser Effekt durch Zusätze von Calciumsalzen, insbesondere Calciumseifen, zurückdrängen, so daß man zu verbesserten Langzeitstabilitäten kommt, es besteht jedoch immer noch ein Bedürfnis an Stabilisatorsystemen, mit denen sich noch bessere Langzeigstabilitäten bei guten Anfangsfarben, insbesondere bei PVC-Massen, erhalten lassen. Diese Aufgabe wird durch die erfindungsgemäß bereitgestellten Metallsalze der 2,2-Dimethylolpropionsäure der obigen allgemeinen Formel gelöst.

Mit den Metallsalzen der 2,2-Dimethylolpropionsäure lassen sich die üblichen, stabilisierungsbedürftigen thermoplastischen Kunststoffe stabilisieren, insbesondere PVC, PVC-Polymerisate und andere chlorhaltige Vinylpolymerisate und -copolymerisate, insbesondere Suspensions-, Masse- und Emulsions-PVC.

Die erfindungsgemäß bereitgestellten Metallsalze der 2,2-Dimethylolpropionsäure der obigen allgemeinen Formel sind neue Verbindungen; bisher sind lediglich die entsprechenden Bleisalze (als 3-basisches Bleidimethylolpropionat) bekannt geworden, vgl. EP-A 0 313 113. Das 3-basische Bleisalz kann als Stabilisator für halogenhaltige Vinylpolymerisate eingesetzt werden.

Die Metallsalze der 2,2-Dimethylolpropionsäure der Erfindung können erhalten werden, indem man 2,2-Dimethylolpropionsäure in wäßriger Lösung, gegebenenfalls in Anwesenheit von mit Wasser mischbaren organischen Hilfslösemitteln wie C₁-C₄-Alkanolen, Tetrahydrofuran oder dergleichen, mit äquivalenten Mengen basischer Verbindungen der entsprechenden Metalle, insbesondere Oxiden, Hydroxiden und Carbonaten, oder mit wasserlöslichen Salzen dieser Metalle, insbesondere Acetaten, umsetzt und die Metallsalze der 2,2-Dimethylolpropionsäure isoliert.

Die Erfindung betrifft weiterhin einen Stabilisator für thermoplastische Kunststoffe, insbesondere für Massen auf Basis von PVC bzw. Copolymeren desselben, enthaltend mindestens ein Metallsalz der 2,2-Dimethylolpropionsäure der obigen Formel I, in der M ein Metall aus der von Magnesium, Calcium und Zink gebildeten Gruppe bedeutet.

Obwohl bereits die einzelnen Metallsalze der 2,2-Dimethylolpropionsäure der Erfindung eine stabilisierende Wirkung in thermoplastischen Kunststoffen enfalten, werden sie bevorzugt in Kombination mit weiteren, stabilisierenden Metallsalzen der 2,2-Dimethylolpropionsäure und/oder einer gesättigten oder ungesättigten Fettsäure mit 8 bis 34 Kohlenstoffatomen eingesetzt. Besonders bevorzugt ist dabei insbesondere ein Gemisch von Calcium- und Zinksalzen der 2,2-Dimethylolpropionsäure; es lassen sich jedoch auch gute Stabilisierungsergebnisse mit Gemischen der entsprechenden Magnesium- und Zinksalze erhalten.

Gemäß einer weiteren vorteilhaften Ausführungsform betrifft die Erfindung einen Stabilisator für thermoplastische Kunststoffe, insbesondere für Massen auf Basis von PVC bzw. Copolymeren desselben, enthaltend
a) Metallsalze der 2.2-Dimethylolpropionsäure der allgemeinen Formel II

   [CH₃ - C(CH₂OH)₂ - COO⁻]₂ (M')²⁺ (II)

   in der M ein Metall aus der von Magnesium, Calcium und Zink gebildeten Gruppe bedeutet, sowie
b) Metallsalze von Fettsäuren der allgemeinen Formel III

   (R - COO⁻)₂ (M'')²⁺ (III)

   in der R ein geradkettiger oder verzweigter, gesättigter oder ungesättigter Kohlenwasserstoffrest mit 7 bis 33 Kohlenstoffatomen bedeutet, M'' ein Metall aus der von Magnesium, Calcium und Zink gebildeten Gruppe bedeutet und M'' ungleich M' ist.

Die vorstehend genannten Metallsalze von Fettsäuren sind von Fettsäuren synthetischer und/oder natürlicher Herkunft abgeleitet, die zur Herstellung der Metallsalze auch in Form ihrer technischen Gemische eingesetzt werden können. Typische Beispiele für derartige Fettsäuren sind Capryl-, Caprin-, Undecylen-, Laurin-, Myristin-, Palmitin-, Palmitolein-, Stearin-, Isostearin-, Öl-, Arachidin-, Eruca-, Behen- und Montansäuren.

Gemäß einer weiteren bevorzugten Ausführungsform der Erfindung bedeutet M' in der Formel II Zink und M'' in der allgemeinen Formel III Calcium; ebenfalls bevorzugt ist die Bedeutung M' = Calcium und/oder Magnesium und M'' = Zink.

Besonders vorteilhafte Stabilisatoren enthalten Metallsalze der allgemeinen Formel III, in der R ein geradkettiger Alkyl- oder ein Alkenylrest mit 10 bis 21 Kohlenstoffatomen ist; typische Vertreter der Fettsäuren, die diesen Metallsalzen zugrunde liegen, ergeben sich aus der obigen Aufstellung.

Besonders bevorzugt sind weiterhin Stabilisatoren der Erfindung, die einen Gehalt an Calcium- oder Magnesium- bis (2,2-dimethylolpropionat) und Salzen des Zinks mit Fettsäuren aus der von Undecylen-, Stearin- und Ölsäure gebildeten Gruppe aufweisen, weiterhin solche mit einem Gehalt an Zink- bis (2,2-dimethylolpropionat) und Salzen des Calciums mit Fettsäuren aus der von Undecylen-, Stearin- und Ölsäure gebildeten Gruppe.

Gemäß einer weiteren vorteilhaften Ausführungsform der Erfindung beträgt in den Stabilisatorkombinationen das Gewichtsverhältnis von Calcium- bzw. Magnesium- zu Zinksalzen 5:1 bis 1:3, jeweils berechnet als metallisches Calcium bzw. Magnesium und Zink.

Den Stabilisatoren der Erfindung können auch weitere, dem Fachmann geläufige Additive zugesetzt werden. Typische Beispiele hierfür sind Kohlenwasserstoffwachse, insbesondere Paraffin- bzw. Polyethylenwachse mit Schmelzpunkten zwischen 52 und 110, insbesondere 70 bis 80°C, Ester und/oder Partialester gesättigter, geradkettiger C₁₂-C₃₄-Monocarbonsäuren, die z.B. in DE-A 26 42 509 und DE-A 36 43 968 beschrieben sind, Natriumalumosilikate, die z.B. in DE-A 29 41 596 beschrieben sind, Antioxidationsmittel, z.B. gemäß DE-A 26 42 509, sekundäre oder tertiäre Ester der phosphorigen Säure mit C₈-C₂₂-Monoalkanolen, Phenol und/oder C₆-C₁₂-alkylsubstituiertem Phenol und gegebenenfalls Polyolen, die 2 oder mehr als 2 Kohlenstoffatome und 2 oder mehr als 2 Hydroxylgruppen aufweisen, die z.B. in DE-A 38 11 493 und US-B 2 997 454 beschrieben sind, epoxidierte Triglyceride ungesättigter Fettsäuren und deren Hydrierungsprodukte, vgl. GB-B 1 020 866 und DE-A 34 20 226, sowie weiterhin übliche, die Rheologie bzw. die Verarbeitbarkeit der Polymerisate beeinflussende Zusätze wie übliche Flow-improver, oxidierte Polyethylenwachse, Wachsester und Hydroxystearinsäuren.

Bevorzugt ist weiterhin ein Stabilisator der Erfindung, der zusätzlich in üblichen Mengen
(a) Kohlenwasserstoffwachse,
(b) Partialester gesättigter, geradkettiger C₁₂-C₃₄-Monocarbonsäuren mit 2 oder mehr als 2 Kohlenstoffatome und 2 oder mehr als 2 Hydroxygruppen aufweisenden Polyolen, die im Zahlenmittel mindestens eine freie Hydroxylgruppe aufweisen,
sowie gegebenenfalls
(c) übliche Additive wie Fettsäuren mit 8 bis 22 Kohlenstoffatomen, Costabilisatoren sowie die Rheologie bzw. die Verarbeitbarkeit der thermoplastischen Kunststoffe beeinflussende Zusätze
enthält.

Die erfindungsgemäßen Stabilisatoren und Stabilisatorgemische werden in der Regel vor der verformenden Verarbeitung mit dem thermoplastischen Kunststoff mechanisch vermischt. In bestimmten Fällen kann es von Vorteil sein, wenn die Stabilisatoren oder Stabilisatorgemische bereits im Verlauf des Polymerisationsverfahrens, insbesondere im Verlauf der Suspensions- oder Emulsionspolymerisation, in die thermoplastischen Kunststoffmassen eingebracht werden.

Die Erfindung betrifft weiterhin die Verwendung von Magnesium-, Calcium und/oder Zinksalzen der 2,2-Dimethylolpropionsäure als Additive für thermoplastische Kunststoffe, insbesondere als Stabilisatoren für Massen auf Basis von PVC bzw. Copolymeren derselben, und zwar vorzugsweise in einer Menge im Bereich von 0,1 bis 5, insbesondere von 0,3 bis 4 Gew.-%, bezogen auf zu stabilisierende thermoplastische Kunststoffe.

Die Erfindung betrifft weiterhin stabilisierte thermoplastische Kunststoffe, die die Metallsalze bzw. die Stabilisatoren der Erfindung enthalten, d.h. stabilisierte thermoplastische Kunststoffe, insbesondere auf Basis von PVC bzw. Copolymeren desselben, enthaltend Metallsalze der 2,2-Dimethylolpropionsäure der allgemeinen Formel II

[CH₃ - C(CH₂OH)₂ - COO⁻]₂ (M')²⁺ (II)

in der M' wie oben definiert ist, sowie gegebenenfalls Metallsalze von Fettsäuren der allgemeinen Formel III

(R - COO⁻)₂ (M'')²⁺ (III)

in der M'' und R wie oben definiert sind, in einer Gesamtmenge im Bereich von 0,1 bis 5, insbesondere von 0,3 bis 4 Gew.-%, bezogen auf zu stabilisierende thermoplastische Kunststoffe.

Die stabilisierten Kunststoffe der Erfindung enthalten bevorzugt weitere Zusätze, z.B. die oben erläuterten, dem Fachmann geläufigen Additive.

Die Erfindung betrifft weiterhin ein Verfahren zur Herstellung von stabilisierten, thermoplastischen Kunststoffen, insbesondere auf Basis von PVC bzw. Copolymeren desselben, gemäß dem man den thermoplastischen Kunststoffen Metallsalze der 2,2-Dimethylolpropionsäure bzw. Stabilisatoren mit den oben genannten Merkmalen zusetzt. Gemäß einer bevorzugten Ausführungsform des Verfahrens setzt man den thermoplastischen Kunststoffen die Metallsalze der 2,2-Dimethylolpropionsäure bzw. die Stabilisatoren in einer Menge zu, daß sie die Metallsalze der 2,2-Dimethylolpropionsäure der allgemeinen Formel II, in der M' wie oben definiert ist, und gegebenenfalls Metallsalze von Fettsäuren der allgemeinen Formel III, in der M'' und R wie oben definiert sind, in einer Gesamtmenge von 0,1 bis 5, insbesondere von 0,3 bis 4 Gew.-%, bezogen auf zu stabilisierenden thermoplastischen Kunststoff, enthalten.

Die Erfindung wird im folgenden anhand von bevorzugten Ausführungsbeispielen näher erläutert.

Im folgenden wird die Abkürzung DMP für die 2,2-Dimethylolpropionsäure bzw. zusammen mit dem entsprechenden chemischen Symbol für die Metallkomponente für die Salze derselben verwendet.

### Herstellung der erfindungsgemäßen DMP-Metallsalze.

Zur Herstellung der DMP-Metallsalze wurde DMP in einem Gemisch von Wasser/Isopropanol oder Wasser/Tetrahydrofuran (Volumenverhältnis 1:1) gelöst und in einem Becherglas vorgelegt. In diese Lösung wurde die Metallverbindung in Form ihres Acetats bzw. ihres Hydroxids, gelöst in Wasser, gegeben. Anschließend wurde ca. 1 h bei 80 bis 90°C gerührt. Die erhaltene wäßrige Lösung bzw. Suspension wurde anschließend bis zur Gewichtskonstanz getrocknet.

### Beispiel 1.

### Herstellung von Ca-DMP.

16,1 g DMP (0,12 mol) und 9,5 g Calciumacetat (0,06 mol) wurden in insgesamt 100 ml Wasser und 100 ml Tetrahydrofuran wie oben beschrieben umgesetzt. Man erhielt 16,5 g eines farblosen, festen Produkts mit einem Ca-Gehalt von 12,1 Gew.-% (Theorie: 12,1 Gew.-%).

### Beispiel 2.

### Zn-DMP.

16,1 g DMP (0,12 mol) und 13,1 g Zinkacetat (0,06 mol) wurden in insgesamt 100 ml Wasser und 100 ml THF wie oben beschrieben umgesetzt. Man erhielt 19,3 g eines farblosen, festen Produkts mit einem Zinkgehalt von 20,6 Gew.-% (Theorie: 19,6 Gew.-%).

### Beispiel 3.

### Mg-DMP.

16,1 g DMP (0,12 mol) und 12,9 g Magnesiumacetat.4H₂O (0,06 mol) wurden in insgesamt 100 ml Wasser und 100 ml THF wie oben beschrieben umgesetzt. Man erhielt 16,6 g eines farblosen, festen Produkts mit einem Mg-Gehalt von 7,1 Gew.-% (Theorie: 8,37 Gew.-%).

### B. Herstellung erfindungsgemäß Ca/Zn- bzw. Mg/Zn-stabilisierter PVC-Formmassen.

Die erfindungsgemäß stabilisierten Kunststoffmassen auf Basis von PVC können hergestellt werden, indem man alle Komponenten wie PVC-Pulver, Gleitmittel und Stabilisatoren bzw. Costabilisatoren in einem hochtourigen Mischer mit Kühl- bzw. Heizeinrichtung bis 90°C vermischt und unter weiterem Rühren auf ca. 40°C abkühlt.

Eine abweichende Mischtechnik, die bei der Herstellung der im folgenden beschriebenen, Calciumseifen enthaltenden Rezepturen angewendet wurde, besteht darin, daß alle Komponenten der Rezeptur bis auf die Calciumseifen zunächst wie vorstehend beschrieben vermischt werden; die Calciumseifen werden dann nachträglich in einem langsam laufenden Mischer bei Raumtemperatur zugemischt.

Die für sämtliche der nachstehend beschriebenen stabilisierten PVC-Formmassen übereinstimmende Grundrezeptur ist aus Tabelle 1 ersichtlich. Die Art und Menge der den PVC-Massen zugesetzten Calcium- bzw. Magnesiumsalze und der in Kombination mit diesen zugesetzten Zinksalze (in Gew.-Teilen, bezogen auf die Grundrezeptur gemäß Tabelle 1) ergibt sich aus Tabelle 2.

Die erfindungsgemäß stabilisierten PVC-Formmassen wurden zur Ermittlung ihrer statischen Stabilität auf einem Laborwalzwerk der Abmessung 450 x 220 mm (Fa. Berstorff) bei einer Walzentemperatur von 170°C und einer Walzendrehzahl von 12,5 U/min im Gleichlauf im Verlauf von 5 min zu Prüffellen verarbeitet. Die ca. 0,5 mm dicken Felle wurden zu rechteckigen Prüfkörpern (15 x 20 mm) zerschnitten und nach DIN 53418 in einem Trockenschrank mit rotierenden Horden bei 180°C thermisch belastet. Die dabei erhaltenen Ergebnisse sind ebenfalls aus Tabelle 2 ersichtlich, und zwar aus den in Klammern gesetzten Werten (in min) für die Anfangsstabilität (erste Zahlenangabe), d.h. die Zeit bis zur ersten wahrnehmbaren Verfärbung, und für den Stabilitätsabbruch (zweite Zahlenangabe), d.h. die Zeit, nach der der Test wegen zu starker Verfärbung abgebrochen wurde.

In der Tabelle 2 ist weiterhin ein Vergleichsversuch mit einer Calciumstearat/Zinkstearat-Stabilisatorkombination (Nr. 7) gemäß dem Stand der Technik enthalten; es zeigt sich, daß mit den Stabilisatorkombinationen der Erfindung Verbesserungen sowohl hinsichtlich der Anfangsstabilität als auch des Stabilitätsabbruchs erhalten wurden.

**Tabelle 1**

| Grundrezeptur: | |
|---|---|
| 100 Gew.-Teile | Suspensions-PVC, K-Wert = 70 |
| 0,5 Gew.-Teile | Pentaerythritester einer gesättigten, technischen C16-C18-Fettsäure (Molverhältnis von Pentaerythrit zu Fettsäure 1:1,8) |
| 0,2 Gew.-Teile | Paraffinwachs |
| 0,2 Gew.-Teile | technische Stearinsäure (45 % C16, 47 % C18) |

**Tabelle 2**

| Stabilisatorkombination | | GT¹ a) | GT¹ b) | Anfangsstabilit_t (min) | Stabilit_tsabbruch (min) |
|---|---|---|---|---|---|
| 1. | a) Ca-DMP | 1,0 | 0,5 | 30 | 60 |
| | b) Zn-Undecylonat | 1,5 | 0,5 | 30 | 70 |
| | | 1,0 | 1,5 | 30 | 50 |
| | | 2,0 | 1,0 | 40 | >60 |
| 2. | a) Ca-DMP | 1,0 | 0,5 | 20 | 70 |
| | b) Zn-Stearat | 1,5 | 0,5 | 20 | 80 |
| | | 1,0 | 1,5 | 10 | 50 |
| 3. | a) Ca-DMP | 1,0 | 0,5 | 10 | 60 |
| | b) Zn-Oleat | 1,5 | 0,5 | 10 | 60 |
| | | 1,0 | 1,5 | 20 | 50 |
| 4. | a) Ca-Undecylenat | 1,0 | 1,5 | 20 | 60 |
| | b) Zn-DMP | | | | |
| 5. | a) Ca-Stearat | 1,0 | 1,5 | 20 | 40 |
| | b) Zn-DMP | | | | |
| 6. | a) Mg-DMP | 1,5 | 0,5 | 10 | 70 |
| | b) Zn-Stearat | | | | |
| 7. | a) Ca-Stearat | 1,0 | 0,5 | 0 | 30-40 ²⁾ |
| | b) Zn-Stearat | | | | |

| | | | | | |
|---|---|---|---|---|---|
| 1) Gewichtsteile auf 100 Gew.-Teile Suspensions-PVC | | | | | |
| 2) Vergleichsversuch | | | | | |

## Patentansprüche

1. Metallsalze der 2,2-Dimethylolpropionsäure der allgemeinen Formel I
[CH₃ - C(CH₂OH)₂ - COO⁻]₂ M²⁺ (I)
in der M ein Metall aus der von Magnesium, Calcium und Zink gebildeten Gruppe bedeutet.

2. Stabilisator für thermoplastische Kunststoffe, insbesondere für Massen auf Basis von PVC bzw. Copolymeren desselben, enthaltend mindestens ein Metallsalz der 2,2-Dimethylolpropionsäure der allgemeinen Formel I
[CH₃ - C(CH₂OH)₂ - COO⁻]₂ M²⁺ (I)
in der M ein Metall aus der von Magnesium, Calcium und Zink gebildeten Gruppe bedeutet.

3. Stabilisator nach Anspruch 2, enthaltend ein Gemisch von Calcium- oder Magnesium- und Zinksalzen der 2,2-Dimethylolpropionsäure.

4. Stabilisator für thermoplastische Kunststoffe, insbesondere für Massen auf Basis von PVC bzw. Copolymeren desselben, enthaltend
a) Metallsalze der 2.2-Dimethylolpropionsäure der allgemeinen Formel II
[CH₃ - C(CH₂OH)₂ - COO⁻]₂ (M')²⁺ (II)
in der M ein Metall aus der von Magnesium, Calcium und Zink gebildeten Gruppe bedeutet, sowie
b) Metallsalze von Fettsäuren der allgemeinen Formel III
(R - COO⁻)₂ (M'')²⁺ (III)
in der R ein geradkettiger oder verzweigter, gesättigter oder ungesättigter Kohlenwasserstoffrest mit 7 bis 33 Kohlenstoffatomen bedeutet, M'' ein Metall aus der von Magnesium, Calcium und Zink gebildeten Gruppe bedeutet und M'' ungleich M' ist.

5. Stabilisator nach Anspruch 4, dadurch gekennzeichnet, daß M' Zink und M'' Calcium bedeuten.

6. Stabilisator nach Anspruch 4, dadurch gekennzeichnet, daß M' Calcium und/oder Magnesium und M'' Zink bedeuten.

7. Stabilisator nach mindestens einem der Ansprüche 4 bis 6, dadurch gekennzeichnet, daß R ein geradkettiger Alkylrest mit 10 bis 21 Kohlenstoffatomen ist.

8. Stabilisator nach mindestens einem der Ansprüche 4 bis 6, dadurch gekennzeichnet, daß R ein geradkettiger Alkenylrest mit 10 bis 21 Kohlenstoffatomen ist.

9. Stabilisator nach mindestens einem der Ansprüche 4 bis 8, gekennzeichnet durch einen Gehalt an Calcium- oder Magnesium-bis(2,2-dimethylolpropionat) und Salzen des Zinks mit Fettsäuren aus der von Undecylen-, Stearin- und Ölsäure gebildeten Gruppe.

10. Stabilisator nach mindestens einem der Ansprüche 4 bis 8, gekennzeichnet durch einen Gehalt an Zink- bis (2,2-dimethylolpropionat) und Salzen des Calciums mit Fettsäuren aus der von Undecylen-, Stearin- und Ölsäure gebildeten Gruppe.

11. Stabilisator nach mindestens einem der Ansprüche 2 bis 10, gekennzeichnet durch ein Gewichtsverhältnis von Calcium- zu Zinksalzen im Bereich 5:1 bis 1:3 (berechnet als jeweils metallisches Calcium bzw. Magnesium und Zink).

12. Stabilisator nach einem der Ansprüche 2 bis 11, dadurch gekennzeichnet, daß er zusätzlich in üblichen Mengen
a) Kohlenwasserstoffwachse,
b) Partialester gesättigter, geradkettiger C₁₂-C₃₄-Monocarbonsäuren mit 2 oder mehr als 2 Kohlenstoffatome und 2 oder mehr als 2 Hydroxygruppen aufweisenden Polyolen, die im Zahlenmittel mindestens eine freie Hydroxylgruppe aufweisen,
sowie gegebenenfalls
c) übliche Additive wie Fettsäuren mit 8 bis 22 Kohlenstoffatomen, Costabilisatoren sowie die Rheologie bzw. die Verarbeitbarkeit der thermoplastischen Kunststoffe beeinflussende Zusätze
enthält.

13. Verwendung von Magnesium-, Calcium- und/oder Zinksalzen der 2,2-Dimethylolpropionsäure als Additive für thermoplastische Kunststoffe, insbesondere als Stabilisatoren für Massen auf Basis von PVC bzw. Copolymeren desselben.

14. Verwendung nach Anspruch 13, dadurch gekennzeichnet, daß die Magnesium-, Calcium- und/oder Zinksalze den thermoplastischen Kunststoffen in einer Menge im Bereich von 0,1 bis 5, insbesondere von 0,3 bis 4 Gew.-%, bezogen auf zu stabilisierenden Kunststoff, zugesetzt werden.

15. Stabilisierte thermoplastische Kunststoffe, insbesondere auf Basis von PVC bzw. Copolymeren desselben, enthaltend Metallsalze der 2,2-Dimethylolpropionsäure der allgemeinen Formel II
[CH₃ - C(CH₂OH)₂ - COO⁻]₂ (M')²⁺ (II)
in der M' wie oben definiert ist, sowie gegebenenfalls Metallsalze von Fettsäuren der allgemeinen Formel III
(R - COO⁻)₂ (M'')²⁺ (III)
in der M'' und R wie oben definiert sind, in einer Gesamtmenge im Bereich von 0,1 bis 5, insbesondere von 0,3 bis 4 Gew.-%, bezogen auf zu stabilisierenden thermoplastischen Kunststoff.

16. Verfahren zur Herstellung von stabilisierten, thermoplastischen Kunststoffen, insbesondere auf Basis von PVC bzw. Copolymeren desselben, dadurch gekennzeichnet, daß man den thermoplastischen Kunststoffen Metallsalze der 2,2-Dimethylolpropionsäure nach Anspruch 1 bzw. Stabilisatoren gemäß mindestens einem der Ansprüche 2 bis 12 zusetzt.

17. Verfahren nach Anspruch 16, dadurch gekennzeichnet, daß man den thermoplastischen Kunststoffen die Metallsalze der 2,2-Dimethylolpropionsäure bzw. die Stabilisatoren in einer Menge zusetzt, daß sie die Metallsalze der 2,2-Dimethylolpropionsäure der allgemeinen Formel II, in der M' wie oben definiert ist, und gegebenenfalls Metallsalze von Fettsäuren der allgemeinen Formel III, in der M'' und R wie oben definiert sind, in einer Gesamtmenge von 0,1 bis 5, insbesondere von 0,3 bis 4 Gew.-%, bezogen auf zu stabilisierenden thermoplastischen Kunststoff, enthalten.

## Claims

1. Metal salts of 2,2-dimethylol propionic acid corresponding to general formula I
[CH₃ - C(CH₂OH)₂ - COO⁻]₂ M²⁺ (I)
in which M is a metal from the group consisting of magnesium, calcium and zinc.

2. A stabilizer for thermoplastics, more particularly for compounds based on PVC or copolymers thereof, containing at least one metal salt of 2,2-dimethylol propionic acid corresponding to general formula I
[CH₃ - C(CH₂OH)₂ - COO⁻]₂ M²⁺ (I)
in which M is a metal from the group consisting of magnesium, calcium and zinc.

3. A stabilizer as claimed in claim 2 containing a mixture of calcium or magnesium and zinc salts of 2,2-dimethylol propionic acid.

4. A stabilizer for thermoplastics, more particularly for compounds based on PVC or copolymers thereof, containing
a) metal salts of 2,2-dimethylol propionic acid corresponding to general formula II
[CH₃ - C(CH₂OH)₂ - COO⁻]₂ (M')²⁺ (II)
in which M is metal from the group consisting of magnesium, calcium and zinc,
and
b) metal salts of fatty acids corresponding to general formula III
(R - COO⁻)₂ (M'')²⁺ (III)
in which R is a linear or branched, saturated or unsaturated hydrocarbon radical containing 7 to 33 carbon atoms, M'' is a metal from the group consisting of magnesium, calcium and zinc and M'' is different from M'.

5. A stabilizer as claimed in claim 4, characterized in that M' is zinc and M'' is calcium.

6. A stabilizer as claimed in claim 4, characterized in that M' is calcium and/or magnesium and M'' is zinc.

7. A stabilizer as claimed in at least one of claims 4 to 6, characterized in that R is a linear alkyl radical containing 10 to 21 carbon atoms.

8. A stabilizer as claimed in at least one of claims 4 to 6, characterized in that R is a linear alkenyl radical containing 10 to 21 carbon atoms.

9. A stabilizer as claimed in at least one of claims 4 to 8, characterized by a content of calcium or magnesium bis-(2,2-dimethylol propionate) and salts of zinc with fatty acids from the group consisting of undecylenic acid, stearic acid and oleic acid.

10. A stabilizer as claimed in at least one of claims 4 to 8, characterized by a content of zinc bis-(2,2-dimethylol propionate) and salts of calcium with fatty acids from the group consisting of undecylenic acid, stearic acid and oleic acid.

11. A stabilizer as claimed in at least one of claims 2 to 10, characterized by a ratio by weight of calcium to zinc salts of 5:1 to 1:3 (expressed as metallic calcium or magnesium and zinc).

12. A stabilizer as claimed in any of claims 2 to 11, characterized in that it additionally contains typical quantities of
(a) hydrocarbon waxes,
(b) partial esters of saturated, linear C₁₂₋₃₄ monocarboxylic acids with polyols containing 2 or more than 2 carbon atoms and 2 or more than 2 hydroxy groups which, on a number average, contain at least one free hydroxyl group
and, optionally,
(c) typical additives, such as C₈₋₂₂ fatty acids, co-stabilizers and additives which influence the rheology or processability of the thermoplastics.

13. The use of magnesium, calcium and/or zinc salts of 2,2-dimethylol propionic acid as additives for thermoplastics, more particularly as stabilizers for compounds based on PVC or copolymers thereof.

14. The use claimed in claim 13, characterized in that the magnesium, calcium and/or zinc salts are added to the thermoplastics in a quantity of 0.1 to 5% by weight and, more particularly, in a quantity of 0.3 to 4% by weight, based on thermoplastic to be stabilized.

15. Stabilized thermoplastics, more particularly based on PVC or copolymers thereof, containing metal salts of 2,2-dimethylol propionic acid corresponding to general formula II
[CH₃ - C(CH₂OH)₂ - COO⁻]₂ (M')²⁺ (II)
in which M' is as defined above,
and optionally metal salts of fatty acids corresponding to general formula III
(R - COO⁻)₂ (M'')²⁺ (III)
in which M'' and R are as defined above,
in a total quantity of 0.1 to 5% by weight and, more particularly, 0.3 to 4% by weight, based on thermoplastic to be stabilized.

16. A process for the production of stabilized thermoplastics, more particularly based on PVC or copolymers thereof, characterized in that metal salts of 2,2-dimethylol propionic acid according to claim 1 or stabilizers according to at least one of claims 2 to 12 are added to the thermoplastics.

17. A process as claimed in claim 16, characterized in that the metal salts of 2,2-dimethylol propionic acid or the stabilizers are added to the thermoplastics in such a quantity that they contain the metal salts of 2,2-dimethylol propionic acid corresponding to general formula II, in which M' is as defined above, and optionally metal salts of fatty acids corresponding to general formula III, in which M'' and R are as defined above, in a total quantity of 0.1 to 5% by weight and, more particularly, 0.3 to 4% by weight, based on thermoplastic to be stabilized.

## Revendications

1. Sels métalliques de l'acide 2,2-diméthylolpropionique de la formule générale I
[CH₃ - C(CH₂OH)₂ - COO⁻]₂ M²⁺ (I)
dans laquelle M représente un métal faisant partie du groupe constitué du magnésium, du calcium et du zinc.

2. Stabilisant pour les thermoplastiques, en particulier pour les masses à base de PVC ou de copolymères de celui-ci, renfermant au moins un sel métallique de l'acide 2,2-diméthylolpropionique de la formule générale I
[CH₃ - C(CH₂OH)₂ - COO⁻]₂ M²⁺ (I)
dans laquelle M représente un métal faisant partie du groupe constitué du magnésium, du calcium et du zinc.

3. Stabilisant selon la revendication 2, renfermant un mélange de sels de calcium ou de magnésium et de zinc de l'acide 2,2-diméthylolpropionique.

4. Stabilisant pour les thermoplastiques, en particulier pour les masses à base de PVC ou les copolymères de celui-ci, renfermant
a) des sels métalliques de l'acide 2,2-diméthylolpropionique de la formule générale II
[CH₃ - C(CH₂OH)₂ - COO⁻]₂ (M')²⁺ (II)
dans laquelle M' représente un métal faisant partie du groupe constitué du magnésium, du calcium et du zinc, ainsi que
b) des sels métalliques d'acides gras de la formule générale III
(R - COO⁻)₂ (M'')²⁺ (III)
dans laquelle R représente un radical hydrocarbure saturé ou insaturé, à chaîne droite ou ramifiée, comportant 7 à 33 atomes de carbone, M'' correspond à un métal faisant partie du groupe constitué du magnésium, du calcium et du zinc et M'' est différent de M'.

5. Stabilisant selon la revendication 4, caractérisé en ce que M' représente le zinc et M'', le calcium.

6. Stabilisant selon la revendication 4, caractérisé en ce que M' représente le calcium et/ou le magnésium et M'', le zinc.

7. Stabilisant selon au moins une des revendications 4 à 6, caractérisé en ce que R est un radical alkyle à chaîne droite comportant 10 à 21 atomes de carbone.

8. Stabilisant selon au moins une des revendications 4 à 6, caractérisé en ce que R est un radical alcényle à chaîne droite comportant 10 à 21 atomes de carbone.

9. Stabilisant selon au moins une des revendications 4 à 8, caractérisé par une teneur en bis(2,2-diméthylolpropionate) de calcium ou de magnésium et en sels de zinc avec des acides gras faisant partie du groupe constitué des acides undécylénique, stéarique et oléique.

10. Stabilisant selon au moins une des revendications 4 à 8, caractérisé par une teneur en bis(2,2-diméthylolpropionate) de zinc et en sels de calcium avec des acides gras faisant partie du groupe constitué des acides undécylénique, stéarique et oléique.

11. Stabilisant selon au moins une des revendications 2 à 10, caractérisé par un rapport pondéral entre les sels de calcium et de zinc se situant dans l'intervalle de 5:1 à 1:3 (calculé chaque fois comme calcium ou magnésium et zinc métallique(s)).

12. Stabilisant selon au moins une des revendications 2 à 11, caractérisé en ce qu'il renferme en plus dans les quantités usuelles
a) des cires d'hydrocarbures,
b) des esters partiels d'acides monocarboxyliques en C₁₂ à C₃₄ à chaîne droite, saturés avec des polyols possédant au moins 2 atomes de carbones et au moins 2 groupes hydroxyle, dont la moyenne numérique présente au moins un groupe hydroxyle libre,
ainsi que le cas échéant,
c) des additifs usuels tels des acides gras comportant 8 à 22 atomes de carbone, des costabilisants ainsi que des additifs influençant la rhéologie ou l'ouvrabilité des thermoplastiques.

13. Utilisation de sels de magnésium, de calcium et/ou de zinc de l'acide 2,2-diméthylolpropionique comme additifs pour les thermoplastiques, en particulier comme stabilisants pour les masses à base de PVC ou de copolymères de celui-ci.

14. Utilisation selon la revendication 13, caractérisée en ce que les sels de magnésium, de calcium et/ou de zinc sont ajoutés aux thermoplastiques dans une proportion comprise dans l'intervalle de 0,1 à 5, en particulier de 0,3 à 0,4 % en poids par rapport au thermoplastique à stabiliser.

15. Thermoplastiques stabilisés, en particulier à base de PVC ou de copolymères de celui-ci, renfermant des sels métalliques de l'acide 2,2-diméthylolpropionique de la formule générale II
[CH₃ - C(CH₂OH)₂ - COO⁻]₂ (M')²⁺ (II)
dans laquelle M' est comme défini ci-avant, ainsi que le cas échéant des sels métalliques d'acides gras de la formule générale III
(R - COO⁻)₂ (M'')²⁺ (III)
dans laquelle M'' et R sont comme définis ci-avant, dans une proportion totale se situant dans l'intervalle de 0,1 à 5, en particulier de 0,3 à 4 % en poids, par rapport au thermoplastique à stabiliser.

16. Procédé de fabrication de thermoplastiques stabilisés, en particulier à base de PVC ou de copolymères de celui-ci, caractérisé en ce que l'on ajoute aux thermoplastiques des sels métalliques de l'acide 2,2-diméthylolpropionique selon la revendication 1 ou des stabilisants selon au moins une des revendications 2 à 12.

17. Procédé selon la revendication 16, caractérisé en ce que l'on ajoute aux thermoplastiques les sels métalliques de l'acide 2,2-diméthylolpropionique ou les stabilisants en quantité telle qu'ils contiennent les sels métalliques de l'acide 2,2-diméthylolpropionique de la formule II, dans laquelle M' est comme défini ci-avant et, le cas échéant, des sels métalliques d'acides gras de la formule générale III, dans laquelle M'' et R sont comme définis ci-avant, dans une proportion totale de 0,1 à 5, en particulier de 0,3 à 4 % en poids, par rapport au thermoplastique à stabiliser.
